(19) Europäisches Patentamt
European Patent Office
Office européen des brevets

(11) **EP 2 263 075 B1**

(12) **EUROPÄISCHE PATENTSCHRIFT**

(45) Veröffentlichungstag und Bekanntmachung des Hinweises auf die Patenterteilung:
**01.02.2012 Patentblatt 2012/05**

(21) Anmeldenummer: **09731036.1**

(22) Anmeldetag: **31.03.2009**

(51) Int Cl.:
***G01N 23/04*** *(2006.01)*

(86) Internationale Anmeldenummer:
**PCT/EP2009/002331**

(87) Internationale Veröffentlichungsnummer:
**WO 2009/124676 (15.10.2009 Gazette 2009/42)**

(54) **VERFAHREN ZUM TOMOGRAPHISCHEN VERMESSEN VON MECHANISCHEN WERKSTÜCKEN**

METHOD FOR THE TOMOGRAPHIC MEASUREMENT OF MECHANICAL WORKPIECES

PROCÉDÉ DE MESURE TOMOGRAPHIQUE DE PIÈCES MÉCANIQUES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **07.04.2008 DE 102008018445**

(43) Veröffentlichungstag der Anmeldung:
**22.12.2010 Patentblatt 2010/51**

(73) Patentinhaber: **Carl Zeiss Industrielle Messtechnik GmbH**
**73447 Oberkochen (DE)**

(72) Erfinder: **KUNZMANN, Steffen**
**01279 Dresden (DE)**

(74) Vertreter: **Witte, Weller & Partner**
**Phoenixbau**
**Königstrasse 5**
**70173 Stuttgart (DE)**

(56) Entgegenhaltungen:
**EP-A- 0 292 402          DE-A1-102005 028 420**
**DE-A1-102005 032 686     DE-A1-102005 032 687**
**DE-A1-102005 039 422     US-B1- 6 341 153**

• **KAK AVINASH ET AL: "Principles of Computerized Tomographic Imaging: Chapter 3" PRINCIPLES OF COMPUTERIZED TOMOGRAPHIC IMAGING, IEEE PRESS, NEW YORK, NY, US, 1. Januar 1999 (1999-01-01), Seiten 49-112, XP002403279**

EP 2 263 075 B1

**Beschreibung**

Die Erfindung betrifft ein Verfahren zum tomographischen Vermessen von mechanischen Werkstücken, bei dem das Werkstück und eine das Werkstück durchdringende Strahlung schrittweise relativ zueinander bewegt werden, aus der Wechselwirkung von Werkstück und Strahlung in jeder Bewegungsstellung des Werkstücks ein zweidimensionales Abbild des Werkstücks in einer Abbildungsebene erzeugt und aus den zweidimensionalen Abbildern ein dreidimensionales Ist-Abbild des Werkstücks errechnet wird.

Ein Verfahren der vorstehend genannten Art ist aus DE 10 2005 039 422 A1, US 6341153 B1 und insbesondere aus DE 102005032687 A1 bekannt.

Bei der Herstellung von insbesondere hochwertigen mechanischen Werkstücken ist es erforderlich, das Werkstück nach oder auch während der Herstellung bzw. Bearbeitung zu vermessen, um zu prüfen, ob bestimmte, vorab festgelegte Messpunkte am Werkstück die vorgegebenen Maße innerhalb einer ebenfalls vorgegebenen Toleranz einhalten. Hierzu werden herkömmlich Mehrkoordinaten-Messmaschinen verwendet, die das Werkstück mittels Tastern abtasten und auf diese Weise die Maßhaltigkeit der Werkstückoberflächen prüfen.

Alternativ dazu wird seit einiger Zeit ein Messverfahren zum Vermessen mechanischer Werkstücke eingesetzt, das zunächst in der Medizin als bildgebendes Verfahren zur Untersuchung menschlicher Körper eingesetzt wurde, nämlich das Verfahren der Computertomographie. Bei medizinischen Anwendungen wird der zu untersuchende Körper oder Körperbereich in einer Ebene mittels eines linearen Arrays von Röntgenstrahlungsquellen durchstrahlt. Dem Array steht auf der gegenüberliegenden Seite des Körpers ein entsprechendes Array von Röntgendetektoren gegenüber. Dieses Paar von Arrays wird dann um eine senkrecht zu der Ebene verlaufende Achse um einen Winkelschritt gedreht, und es wird eine weitere Aufnahme hergestellt. Nachdem das Array schrittweise um insgesamt 360° gedreht wurde, wird aus den Einzelaufnahmen ein Querschnittsbild in der Ebene errechnet, das die Dichteverteilung in dieser Ebene wiedergibt. Wenn nun anschließend der Körper und das Paar von Arrays um einen linearen Schritt in der Achse relativ zueinander verschoben werden, kann ein weiteres, unmittelbar benachbartes Querschnittsbild erzeugt werden und aus einer Vielzahl derartiger benachbarter Querschnittsbilder eine dreidimensionale Darstellung des Körpers bzw. Körperbereichs. Dieses Messverfahren ist relativ aufwändig, weil ein menschlicher Körper eine sehr komplexe Dichteverteilung mit in weiten Bereichen variierender Dichte hat und die aufzunehmenden Strukturen in erheblichem Maße unterschiedlich und von unvorhersehbarer Art und Gestalt sein können.

Demgegenüber handelt es sich bei Werkstücken in der Qualitätskontrolle üblicherweise um Objekte mit nur zwei Dichten, nämlich der Dichte des Werkstoffs des Werkstücks und der Dichte von Luft. Ferner sind bei der Qualitätskontrolle die zu untersuchenden Strukturen bekannt und müssen lediglich auf Abweichungen hin erfasst werden.

In der eingangs genannten DE 10 2005 039 422 A1 wird ein im Vergleich zu medizinischen Anwendungen vereinfachtes Computertomographie-Verfahren zum Untersuchen von Werkstücken beschrieben. Bei diesem Verfahren befindet sich ein mechanisches Werkstück auf einem Drehtisch zwischen einer punktförmigen Röntgen-Strahlungsquelle und einem flächigen Detektorarray. Die Drehachse des Drehtischs verläuft dabei im Wesentlichen senkrecht zur Strahlungsrichtung. Das Werkstück wird von der Röntgenstrahlung durchsetzt, und auf dem Detektorarray entsteht ein Schattenbild des Werkstücks. Das Werkstück wird dann auf dem Drehtisch nacheinander, beispielsweise 800 oder 1.200 mal, um einen Winkelschritt gedreht, und es werden weitere Schattenbilder erzeugt. Aus der Vielzahl der Schattenbilder wird dann ein dreidimensionales Abbild des Werkstücks errechnet werden, beispielsweise nach einem Verfahren der Rückprojektion, wie es in der DE 39 24 066 A1 beschrieben ist.

Computertomographische Messplätze dieser Art werden mittlerweile auch kommerziell hergestellt und angeboten, beispielsweise von der Anmelderin unter der Typenbezeichnung "Metrotom" (www.zeiss.de/imt).

Bei den bekannten Messplätzen wird eine so genannte "Rekonstruktion" durchgeführt, d.h. dass ein vollständiges dreidimensionales Abbild der Dichteverteilung innerhalb des Messobjektes (mechanisches Werkstück) mit einer vorbestimmten Auflösung von Volumenelementen (Voxeln) errechnet wird. Um dies mit der für eine Werkstückvermessung ausreichenden Präzision tun zu können, wird eine große Anzahl von Einzelabbildern benötigt, in der Praxis zwischen 360 und 1080, entsprechend Winkelschritten des Drehtischs zwischen 1° und 1/3°. Für jeden Drehschritt wird jedoch eine gewisse Zeit benötigt, um den Drehtisch aus der Ruhelage zu beschleunigen, zu bewegen und wieder bis zum Stillstand abzubremsen. Ferner wird für die Berechnung des dreidimensionalen Abbildes, auch wenn diese bereits während der laufenden Messung gestartet werden kann, eine gewisse Zeit benötigt, so dass man im Ergebnis mit derzeit bekannten Messplätzen für eine komplette Messung zwischen 15 und 30 Minuten benötigt. Diese Messzeit ist in vielen Fällen nicht akzeptabel.

Der Erfindung liegt daher die Aufgabe zugrunde, ein Verfahren der eingangs genannten Art anzugeben, das die vorstehend genannten Nachteile vermeidet. Insbesondere soll ein Verfahren zur Verfügung gestellt werden, das eine wesentlich kürzere Gesamtmesszeit erfordert.

Bei einem Verfahren der eingangs genannten Art wird diese Aufgabe erfindungsgemäß dadurch gelöst, dass aus mindestens zwei in zwei unterschiedlichen Bewegungsstellungen erzeugten zweidimensionalen Abbildern einer innerhalb des Werkstücks vorhandenen, regelmäßigen Ist-Struktur Punkte an einem kontrastreichen Übergang in den zweidimensionalen Abbildern erfasst werden und aus der Lage der Punkte ein dreidimensionaler Ausgleichskörper bestimmt und dieser mit einer vorgegebenen Soll-Struktur verglichen wird.

Die der Erfindung zugrunde liegende Aufgabe wird auf diese Weise vollkommen gelöst.

Es ist nämlich das Verdienst des Erfinders, erkannt zu haben, dass sich die Regelmäßigkeit der Form bestimmter Strukturen in mechanischen Werkstücken (Bohrungen, Nuten usw.), anders als bei natürlichen Objekten in der Medizin, dazu nutzen lässt, die Gesamtmesszeit drastisch zu reduzieren, indem aus verhältnismäßig wenigen Messpunkten in Kenntnis der Regelmäßigkeit der Struktur ein Abbild dieser Struktur hergestellt wird. Es werden nämlich nur einzelne, vorzugsweise rotationssymmetrische Elemente tomographisch oder durch andere Verfahren mit einer zweidimensionalen Projektion aus mehreren Richtungen in einer kalibrierten Aufnahmevorrichtung vermessen und durch einen geometrischen Ausgleichskörper parametrisiert. Diese Vorgehensweise unterscheidet sich von herkömmlichen Verfahren aus dem Stand der Technik, weil dort eine Rekonstruktion der gesamten Dichteverteilung in einer Voxelmatrix stattfindet.

Erfindungsgermäß macht man sich daher die Tatsache zunutze, dass ein großer Teil der vom CT-Anwender im Rekonstruktionsvolumen gesuchten Elemente regelmäßig, insbesondere rotationssymmetrisch ist und diese Elemente sich daher auch in der einzelnen Projektion mit scharfen, kontrastreichen Objektgrenzen abbilden. Diese Objektgrenzen kann man mit Hilfe von Bildverarbeitungsmethoden, beispielsweise mit einer geeigneten Kantenerkennung, aus den einzelnen Projektionen extrahieren. Wenn mehrere Projektionen mit verschiedener, bekannter Orientierung aufgenommen wurden, Können daraus die Position, die Achsrichtung und weitere Formparameter, beispielsweise ein Radius oder ein Kegelwinkel, des dreidimensionalen Elements ermittelt werden.

Bei dem erfindungsgemäßen Verfahren entfällt die herkömmliche komplette Aufnahme aller Projektionsbilder eines CT-Scans. Im Prinzip können bereits aus zwei Projektionen die Parameter der gesuchten dreidimensionalen Struktur grob ermittelt werden.

Erfindungsgemäß entfällt die bislang erforderliche rechenzeitaufwändige CT-Rekonstruktion einer gesamten Voxelmatrix. Erste Versuche haben ergeben, dass sich die Gesamtmesszeit auf diese Weise von den herkömmlichen 15 bis 30 Minuten auf weniger als 1 Minute reduzieren lässt.

Bei einem bevorzugten Ausführungsbeispiel der Erfindung ist die rotationssymmetrische Sollstruktur eine Kugel, ein Zylinder oder ein Kegel.

Bei einer bevorzugten Ausgestaltung des erfindungsgemäßen Verfahrens sind die Strahlungsquelle und die Abbildungsebene für die relative Bewegung raumfest, und das Werkstück wird in vorgegebenen Winkelschritten um eine Drehachse gedreht.

Diese Maßnahme hat den Vorteil, dass hinsichtlich des Messplatzes auf bewährte Komponenten Zurückgegriffen werden kann.

Bei einem weiteren Ausführungsbeispiel werden bei einer kugelförmigen Soll-Struktur die Koordinaten eines Mittelpunktes und ein Radius des Ausgleichskörpers ermittelt.

Diese Maßnahme hat den Vorteil, dass eine grobe Abschätzung des Ausgleichskörpers mit einem Minimum an Parametern und daher mit einem Minimum an Aufwand möglich ist.

Im Falle der beiden vorgenannten Ausführungsbeispiele zeichnet sich eine besonders bevorzugte Weiterbildung dadurch aus, dass zum Ermitteln der Lage des Mittelpunktes des Ausgleichskörpers in folgenden Schritten vorgegangen wird:

a) Ermitteln der Koordinaten von mindestens drei Punkten auf dem Rand des kreisförmigen, zweidimensionalen Abbildes der Ist-Struktur in einer ersten Bewegungsstellung des Werkstücks;

b) Ermitteln eines ersten Mittelpunktes des kreisförmigen Abbildes aus den Koordinaten der drei Punkte;

c) Verdrehen des Werkstücks einerseits und der Strahlungsquelle sowie der Abbildungsebene andererseits relativ zueinander um einen vorgegebenen Winkelschritt von der ersten in eine zweite Bewegungsstellung des Werkstücks;

d) Ermitteln der Koordinaten von mindestens drei Punkten auf dem Rand des bewegten kreisförmigen, zweidimensionalen Abbildes der Ist-Struktur in der zweiten Bewegungsstellung des Werkstücks;

e) Ermitteln eines zweiten Mittelpunktes des bewegten kreisförmigen Abbildes aus den Koordinaten der drei Punkte;

f) Ermitteln zweier Mittelpunktsstrahlen zwischen der Strahlungsquelle und dem ersten bzw. zweiten Mittelpunkt;

g) Ermitteln des Ortes des geringsten Abstandes zwischen den Mittelpunktsstrahlen;

h) Fällen des Verbindungslotes zwischen den Mittelpunktsstrahlen am Ort der geringsten Abstandes; und

i) Ermitteln der Koordinaten des Mittelpunktes des Verbindungslotes als Ort des Mittelpunktes des Ausgleichskörpers.

In Ergänzung dazu ist besonders bevorzugt, wenn zum Ermitteln des Radius ($R_A$) des Ausgleichskörpers in folgenden Schritten vorgegangen wird:

j) Ermitteln eines ersten Abstandes zwischen der Strahlungsquelle und dem Mittelpunkt des kreisförmigen Abbildes;

k) Ermitteln eines zweiten Abstandes zwischen der Strahlungsquelle und dem Mittelpunkt der Ist-Struktur; und

l) Multiplizieren des Radius des kreisförmigen Abbildes mit dem Quotienten aus dem zweiten und dem ersten Abstand.

Diese Maßnahmen haben den Vorteil, dass die erwähnten wenigen Parameter der kugelförmigen Struktur mit wenigen Operationen bestimmt werden können.

Bei einer Weiterbildung des vorgenannten Ausführungsbeispiels werden für eine Mehrzahl der in Schritt a) ermittelten Punkte Tangentialpunkte bestimmt, in denen ein Verbindungsstrahl zwischen der Strahlenquelle und den Punkten einen das Abbild erzeugenden Rand der Ist-Struktur berührt, und der Ausgleichskörper wird in die aus den Punkten gebildete Punktwolke mit den in Schritt i) und l) ermittelten Werten für die Lage des Mittelpunktes und des Radius des Ausgleichskörpers als Startwerte eingepasst.

Diese Maßnahme hat den Vorteil, dass die Genauigkeit der Einpassung erheblich gesteigert werden kann.

Dies gilt noch in größerem Maße, wenn die Punktwolke aus in unterschiedlichen Drehstellungen des Werkstücks ermittelten Tangentialpunkten gebildet wird.

Weitere Vorteile ergeben sich aus der Beschreibung und der beigefügten Zeichnung.

Ausführungsbeispiele der Erfindung sind in der Zeichnung dargestellt und werden in der nachfolgenden Beschreibung näher erläutert. Es zeigen:

Fig. 1A-C: eine technische Zeichnung, in drei orthogonalen Ansichten, eines mechanischen Werkstücks, wie es mit dem erfindungsgemäßen Verfahren vermessen werden kann;

Fig. 2A-B: eine extrem schematisierte Ansicht eines Messplatzes zum Durchführen des erfindungsgemäßen Verfahrens in einer Seitenansicht bzw. einer Draufsicht;

Fig. 3A-B: eine perspektivische Ansicht des Messplatzes von Figur 2A-B, in einer ersten Drehstellung des Werkstücks bzw. in einer zweiten Drehstellung des Werkstücks;

Fig. 3C: eine Darstellung zum Erläutern einer Kreisgleichung;

Fig. 4 eine Darstellung zur Erläuterung des Auffindens von Startparametern; und

Fig. 5 eine Darstellung eines Ausgleichskörpers.

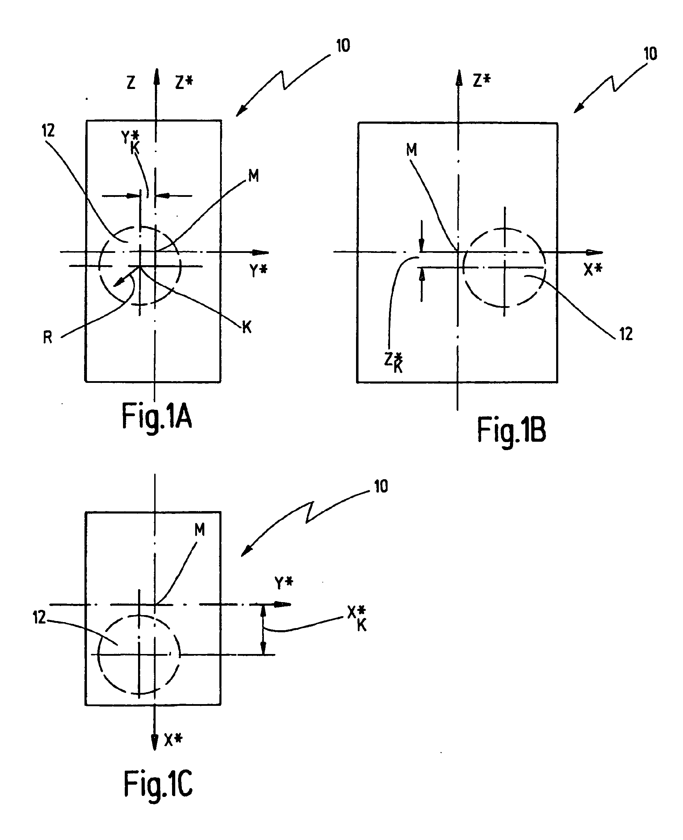

In den Figuren 1A-1C sind drei zueinander senkrechte Ansichten eines mit dem allgemeinen Bezugszeichen 10 versehenen Werkstücks dargestellt. Das Werkstück 10 ist im dargestellten einfachen Beispiel ein quaderförmiger Körper. Das Werkstück 10 besteht aus einem bekannten Werkstoff, der als homogen angenommen wird, also in sich keine Dichteschwankungen aufweist.

Das Werkstück 10 weist eine im dargestellten Ausführungsbeispiel kugelförmige innere Struktur 12 auf, deren Mittelpunkt mit K und deren Radius mit R bezeichnet ist. Die Struktur 12 hat im Vergleich zum übrigen Werkstück 10 andere Eigenschaften, insbesondere eine andere Dichte. Im dargestellten Beispiel kann sie ein kugelförmiger, luftgefüllter Hohlraum im Werkstück 10 sein.

Die Struktur 12 ist eine Ist-Struktur, die in einem beliebigen Herstellungsprozess innerhalb des Werkstücks 10 erzeugt wurde. Die Struktur 12 stimmt daher in der Praxis weder nach ihrer Lage noch nach ihrer Form exakt mit einer von einem Konstrukteur vorgegebenen Soll-Struktur überein, die beispielsweise in einem CAD-File abgespeichert ist. Es ist das Ziel des vorliegenden Verfahrens, festzustellen, in welchem Maße die Ist-Struktur 12 von dieser Soll-Kontur abweicht. Zu diesem Zweck wird die Ist-Struktur 12 vermessen und mit einem optimal eingepassten Ausgleichskörper bestmöglich angenähert. Die charakteristischen Daten des Ausgleichskörpers, im dargestellten Ausführungsbeispiel also die Lage des Mittelpunktes K im Werkstück 10 und der Radius R, werden dann mit den konstruktiv vorgegebenen Daten der Soll-Struktur verglichen. Aus diesem Vergleich wird abgeleitet, ob das Werkstück 10 hinsichtlich der Struktur innerhalb vorgegebener Toleranzen liegt.

Das Werkstück und die innere Struktur können selbstverständlich auch eine andere Form aufweisen. Das Werkstück ist vorzugsweise ein Bauteil einer Maschine oder eines Gerätes von beliebiger Form, in und/oder an dem sich Strukturen von im Wesentlichen rotationssymmetrischer Form befinden, beispielsweise zylinder-förmige, kegelförmige, paraboloide, und sonstige rotationssymmetrische Formen.

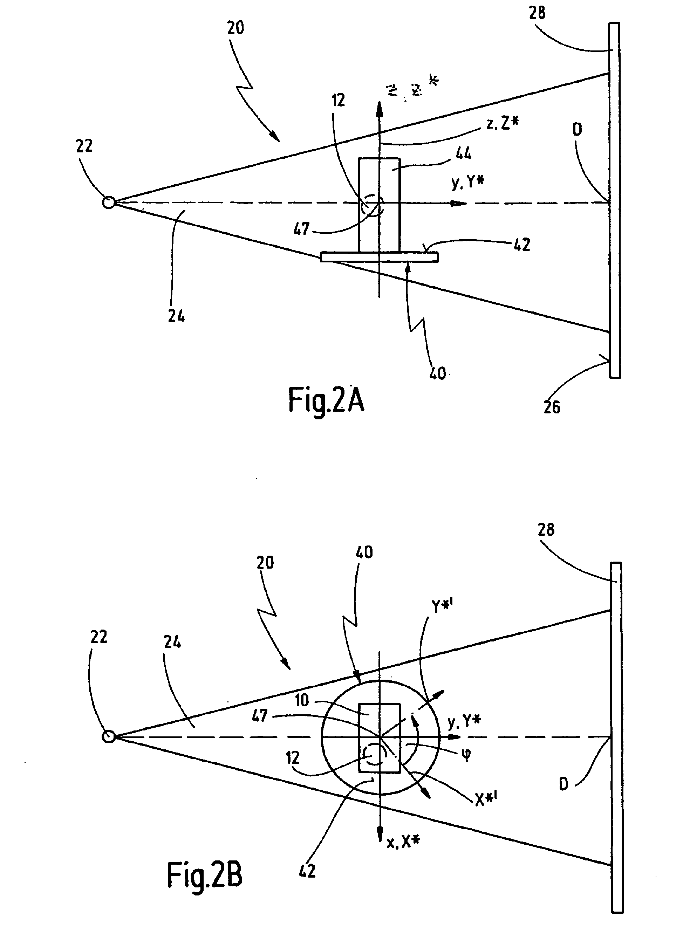

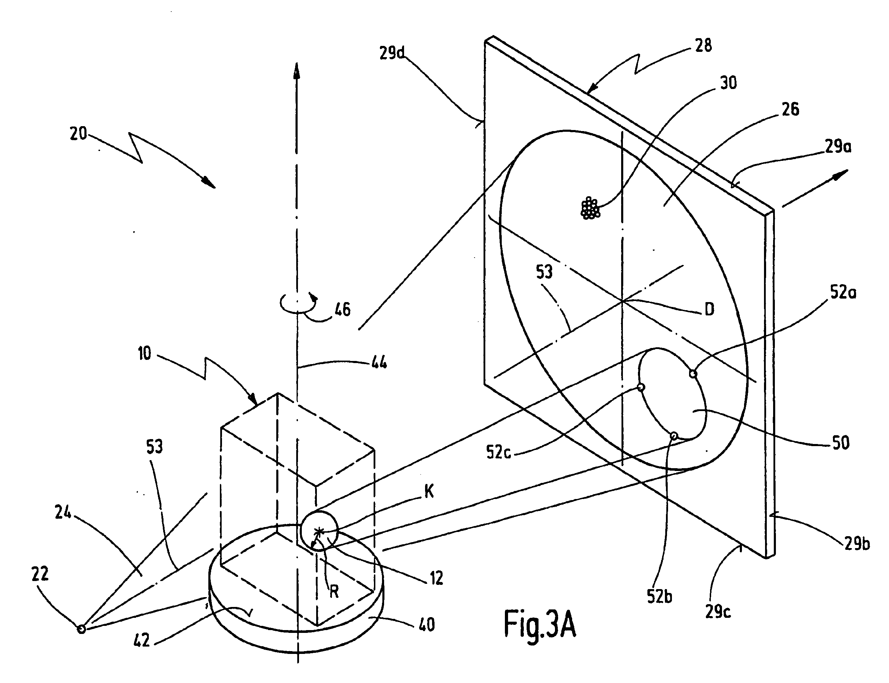

In den Figuren 2A-B und 3A-B ist dargestellt, wie das Werkstück 10 tomographisch vermessen wird.

Das Werkstück 10 wird hierzu in einem Messplatz 20 angeordnet. Der Messplatz 20 enthält eine punktförmige Strahlungsquelle 22, die vorzugsweise als Röntgenquelle ausgebildet ist und die das Werkstück 10 von der Seite her beleuchtet. Die Strahlungsquelle 22 ist raumfest angeordnet und sendet eine kegelförmige Strahlung 24 aus. Die Strahlung 24 durchdringt das Werkstück 10 und fällt auf einen kreisförmigen Bereich 26 eines hinter dem Werkstück 10 angeordneten Detektorarrays 28.

Es versteht sich an dieser Stelle, dass die punktförmige Röntgen-Strahlungsquelle 22 hier nur beispielhaft zu verstehen ist. Selbstverständlich können im Rahmen der vorliegenden Erfindung auch andere Strahlungsquellen, beispielsweise lineare Arrays von Strahlungsquellen, verwendet werden. Weiterhin ist es auch möglich, andere bildgebende Untersuchungsverfahren zu verwenden, beispielsweise die NMR-Tomographie.

Das Detektorarray 28 ist eben und rechteckförmig, vorzugsweise quadratisch, mit Kanten 29a - 29d ausgebildet. Es weist in einer Fläche eine Vielzahl von zueinander benachbart angeordneten Detektoren 30 auf. Im dargestellten Ausführungsbeispiel haben die Detektoren 30 eine Auflösung von 512 x 512 Pixeln mit einem Pixelabstand von 0,1 mm. Es versteht sich jedoch, dass auch andere Auflösungen einsetzbar sind. Ein Mittelpunkt des Detektorarrays 28 ist mit D bezeichnet.

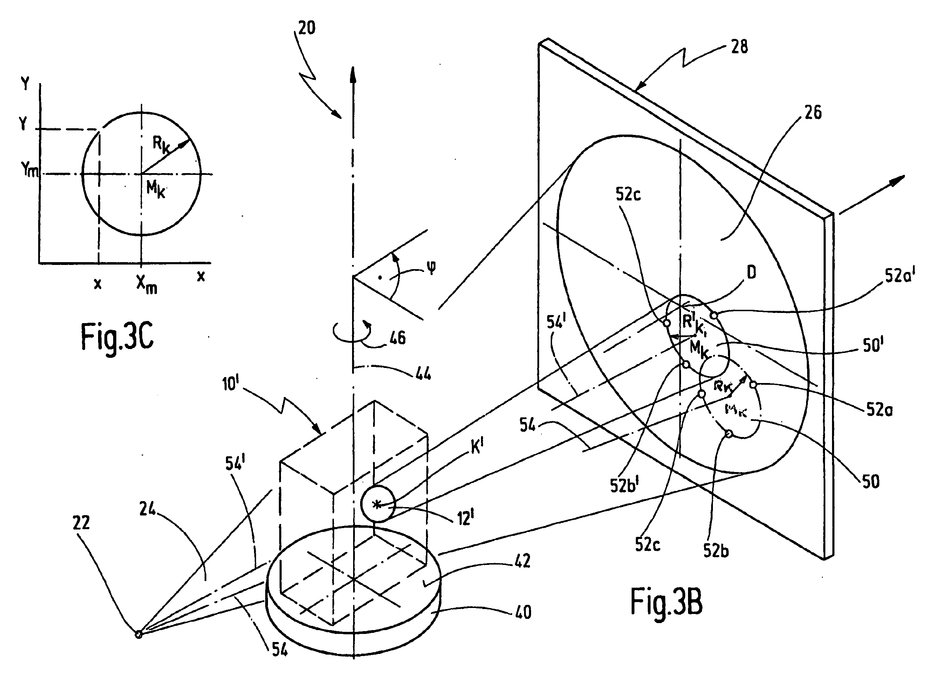

Das Werkstück 10 ist während des Vermessens auf einem Drehtisch 40 angeordnet, vorzugsweise steht es auf einer Oberfläche 42 des Drehtischs 40. Der Drehtisch 40 ist um eine Drehachse 44 drehbar, die vorzugsweise senkrecht zu der Oberfläche 42 verläuft. Ein Drehen des Drehtisches 40 um die Drehachse 44 in Richtung eines Pfeils 46 bewirkt eine Drehung des Werkstücks 10 um einen entsprechenden Drehwinkel $\varphi$. In Figur 3B sind die Bezugszeichen der durch die Drehung bewegten Elemente mit einem Apostroph versehen.

Es versteht sich an dieser Stelle, dass die raumfeste Anordnung der Strahlungsquelle 22 und des Detektorarrays 28 sowie die drehbare Anordnung des Werkstücks 10 ebenfalls nur beispielhaft zu verstehen sind und die vorliegende Erfindung nicht einschränkt. Zum einen kann in kinematischer Umkehr auch das Werkstück feststehen und sich die Strahlungsquelle mit dem Detektorarray drehen. Weiterhin kann die Strahlungsquelle feststehen und nur die Strahlung mit sich ändernder Richtung abgelenkt werden. Schließlich kann anstelle einer Drehbewegung auch eine andere Bewegung gewählt werden.

Die Strahlungsquelle 30 und der Mittelpunkt D des Detektorarrays 28 sind durch eine Verbindungslinie 53 miteinander verbunden, die eine Achse y eines raumfesten Koordinatensystems x, y, z des Messplatzes 20 definiert. Der Ursprung 47 des raumfesten Koordinatensystems x, y, z liegt innerhalb des Drehtischs 40 auf der Drehachse 44. Die Achse z fällt mit der Drehachse 44 zusammen. Im dargestellten Beispiel fällt der Ursprung 47 der Übersichtlichkeit halber mit dem Mittelpunkt M des Werkstücks 10 zusammen.

Das Detektorarray 28 ist derart relativ zum raumfesten Koordinatensystem x, y, z angeordnet, so dass seine Oberfläche in einer Ebene parallel zur x-z-Ebene verläuft und Kanten 29a, 29c parallel zur x-Achse und die Kanten 29b, 29d parallel zur z-Achse verlaufen.

Der Mittelpunkt M des Werkstücks 10 auf dem Drehtisch 40 bzw. der Ursprung 47 des raumfesten Koordinatensystems x, y, z ist im dargestellten Beispiel zugleich der Koordinatenursprung eines rotierenden Koordinatensystems x*, y*, z*, wobei die Achsen z und z* zusammenfallen. Die Achsen x* und y* drehen sich relativ zu den Achsen x und y, wie in Figur 2B mit x*' und y*' veranschaulicht. Der Mittelpunkt K der kugelförmigen Struktur 12 ist im rotierenden Koordinatensystem x*, y*, z* an der Position $x_k^*$, $y_k^*$, $z_k^*$ angeordnet, wie in den Figuren 1A bis 1C gezeigt.

Durch Bestrahlen des Werkstücks 10 mittels der Strahlungsquelle 22 ergibt sich ein Schattenwurf der kugelförmigen Struktur 12 des Werkstücks 10 auf dem Detektorarray 28 in Form eines kreisförmigen zweidimensionalen Abbildes 50 der kugelförmigen Struktur 12. Wenn das Werkstück 10 um einen Winkelschritt um die Drehachse 44 (z*-Achse) gedreht wird, ändert sich die Lage des Abbildes auf dem Detektorarray 28 von 50 nach 50', wie in Figur 3B für einen Drehwinkel $\varphi$ = 90° beispielhaft dargestellt.

Aus einer Mehrzahl von Abbildern 50, 50' ..., die unter verschiedenen Winkeln $\varphi$ aufgenommen wurden, werden unter Verwendung von mindestens drei Messpunkten 52a - 52c, 52a' - 52c', die auf einem Rand der Abbilder 50, 50' liegen, die Position und die Abmessungen der kugelförmige Struktur 12 und damit ein interessierender Bereich des Werkstücks 10 rekonstruiert, wie nachstehend erläutert werden wird.

In einem ersten Schritt werden der Mittelpunkt K, d.h. die Position der kugelförmigen Struktur 12 bezüglich des raumfesten Koordinatensystems x, y, z, sowie der Radius R der Struktur 12 ermittelt. Hierzu werden die Messpunkte 52a - 52c, 52a' - 52c' erfasst. Dabei werden mittels bekannter Methoden der Bildverarbeitung bzw. Bildanalyse die Ränder der Kugelprojektion, d.h. der Abbilder 50, 50', aufgefunden und in eine geeignete Zahl von Messpunkten transformiert. Aus diesen Messpunkten werden zur Vereinfachung drei Punkte ausgewählt, die vorzugsweise in etwa äquidistant über den Umfang des Abbildes verteilt sind.

Allgemein gesprochen werden Punkte an kontrastreichen Übergängen in den Abbildern 50, 50' erfasst, die Abbildungsrändern und Kanten der zu untersuchenden Strukturen entsprechen können.

Zur Ermittlung der Position des Mittelpunktes K und des Radius R der kugelförmigen Struktur 12 werden aus den Koordinaten der jeweiligen drei Messpunkte 52a - 52c, 52a' - 52c' der Abbilder 50, 50' unter Verwendung der bekannten und in Figur 3 C dargestellten Kreisgleichung

$$(x-x_m)^2 + (z-z_m)^2 = R_k^2$$

die Koordinaten $x_m$ und $z_m$ des jeweiligen Kreismittelpunkts $M_k$, $M_k'$ und der Kreisradius $R_k$, $R_k'$ der Abbilder 50 und 50' ermittelt.

Nun werden Mittelpunktsstrahlen 54, 54' von der punktförmigen Strahlungsquelle 22 zu den Mittelpunkten $M_k$ und $M_k$ der Abbilder 50, 50' ermittelt. Der Mittelpunktsstrahl 54' wird dann zur Kompensation der Drehbewegung des Drehtisches 40 um den Winkelschritt φ zurückgedreht, indem die Strahlungsquelle 22 und der Mittelpunkt $M_k'$ rechnerisch um -φ um die Drehachse 44 zurückgedreht werden.

Im Idealfalle würde man auf diese Weise zwei Mittelpunktsstrahlen erhalten, die räumlich zusammenfallen. Trotz höchstpräziser Führungen und Stellsysteme tritt in der Praxis jedoch ein unvermeidbarer Restfehler auf, so dass man als Ergebnis der Rechnung nicht zwei zusammenfallende Geraden erhält, sondern zwei windschiefe Geraden. Es werden nun die Punkte auf diesen beiden Geraden ermittelt, in denen der Abstand zwischen den Geraden minimal ist. Dann wird das Verbindungslot zwischen diesen Punkten errichtet. Der Mittelpunkt der Strecke auf dem Verbindungslot zwischen den beiden Punkten wird dann als beste Näherung für die Lage des Mittelpunktes K im x, y-Koordinatensystem angenommen.

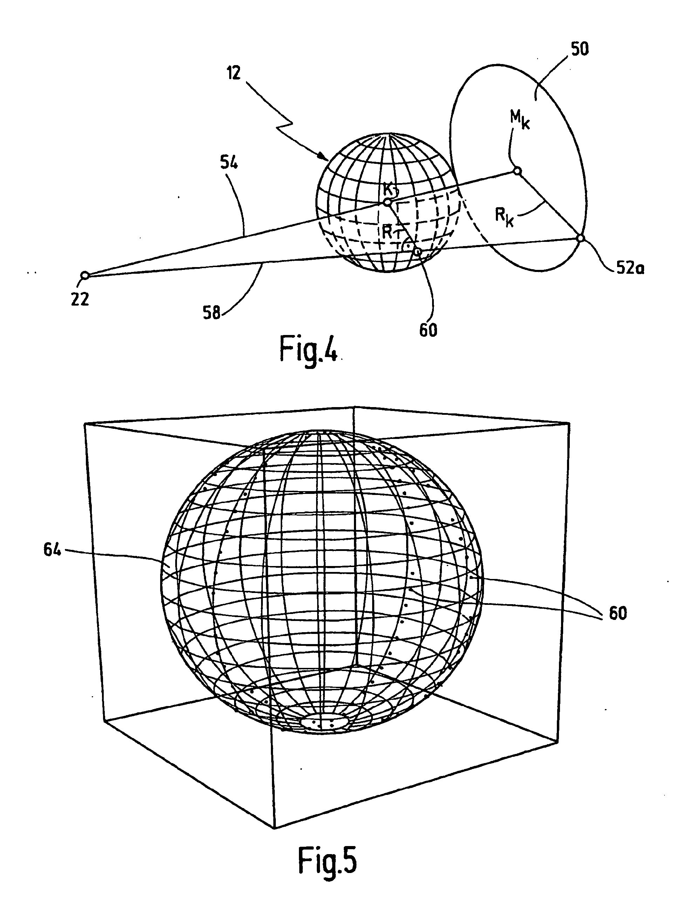

Sodann wird, wie in Figur 4 veranschaulicht, der Radius R der Struktur 12 unter Verwendung des Strahlensatzes ermittelt. Dabei macht man sich die Tatsache zunutze, dass das Verhältnis des Abstandes der Strahlungsquelle 22 vom Mittelpunkt $M_k$ des Abbildes 50 zum Abstand der Strahlungsquelle 22 vom Mittelpunkt K der Struktur 12 gleich dem Verhältnis des Radius $R_k$ des Abbildes 50 zum Radius R der Struktur 12 ist.

Damit liegen Startwerte für einen kugelförmigen Ausgleichskörper fest, nämlich die Position eines Mittelpunktes $K_A$ sowie eines Radius $R_A$. Bereits aus diesen Werten, die sich aus nur zwei Messungen mit unterschiedlicher Projektion des Werkstücks 10 auf das Detektorarray 28 ergeben, lässt sich somit der Ausgleichskörper grob nach Lage und Form bestimmen.

Man erkennt aus Figur 4 ferner, dass jeder Tangentialstrahl 58, der von der Strahlenquelle 22 zu einem Punkt 52 auf dem Rand des Abbildes 50 führt, die Struktur 12 in einem Tangentialpunkt 60 berührt. Die Strecke zwischen dem Mittelpunkt K und dem Tangentialpunkt 60, also der Radius R, steht dabei senkrecht auf dem Tangentialstrahl 58.

Diese Tangentialbedingung kann man heranziehen, um zu jedem Tangentialstrahl 58, der die Struktur 12 entlang eines Umfanges streift, den zugehörigen Tangentialpunkt 60 zu ermitteln. Die so ermittelten Tangentialpunkte 60 können dann für eine Best-Fit-Rechnung (z.B. Least Square Fit) verwendet werden.

Dabei wird vorzugsweise durch Berechnung der Ableitungen in der Bestimmungsgleichung der Tangentialpunkte an den Orten der einzelnen Tangentialpunkte zusammen mit den orthogonalen Abständen der Tangentialpunkte zum Ausgleichskörper über ein Gleichungssystem ein Satz von Verbesserungen der Parameter des Ausgleichskörpers berechnet. Dabei kann das bekannte Verfahren des Least-Square-Fit, aber auch andere (Hüll-Einpassung, Pferch-Einpassung, Tschebyscheff-Einpassung) verwendet werden. Nach jeder Verbesserung der Parameter des Ausgleichskörpers werden die Tangentialpunkte neu bestimmt. Die Optimierung wird so lange iterativ durchgeführt, bis die Verbesserungen einen vorbestimmten Restwert unterschreiten. Damit ist der optimale Ausgleichskörper bestimmt, und die Restabstände der Tangentialpunkte zum Ausgleichskörper können, wie in der 3D-Messtechnik üblich, zum Beispiel zur Analyse der Formabweichungen verwendet werden. Dieses Verfahren ist dem Fachmann bekannt.

Wie bereits oben erwähnt, werden nun die charakteristischen Daten des optimierten Ausgleichskörpers, im dargestellten Ausführungsbeispiel also die Lage des Mittelpunktes K im Werkstück 10 und der Radius R, mit den konstruktiv vorgegebenen Daten der Soll-Struktur verglichen. Aus diesem Vergleich wird eine Gutteil/Schlechtteil-Entscheidung abgeleitet, d.h. festgestellt, ob das Werkstück 10 hinsichtlich der Struktur innerhalb vorgegebener Toleranzen liegt.

Man kann dabei die Genauigkeit weiter dadurch steigern, dass die Tangentialpunkte 60 in mehreren Projek-tionen bzw. Drehstellungen φ des Werkstücks 10 ermittelt werden, beispielsweise in 6 oder 36 Projektionen. Dann wird das Best-Fit-Verfahren auf alle Tangentialpunkte 60 der mehreren Projektionen angewendet.

Figur 5 zeigt dazu ein Beispiel eines optimierten Ausgleichskörpers 64 für eine kugelförmige Struktur, die durch sechs Projektionen unter unterschiedlichen Winkeln in die Tangentialpunkte 60 eingepasst wurde.

Anstelle der Approximation mittels des Best-Fit-Verfahrens können die Ausgleichskörper auch unter Verwendung einer Hüll-, Pferch- oder Tschebytscheff-Approximation ermittelt werden.

Das für einen kugelförmigen Ausgleichskörper beschriebene BestFit-Verfahren kann, wie ebenfalls bereits erwähnt, auch für einen zylinderförmigen oder einen kegelförmigen Ausgleichskörper verwendet werden, wenn die Struktur 12 zylinder- bzw. kegelförmig ist. Die zu bestimmenden Startparameter des Zylinders sind der Zylinderradius sowie die Ausrichtung der Zylinderachse. Im Falle eines Ausgleichskegels sind die Startparameter der Öffnungswinkel des Kegels, die Ausrichtung der Kegelachse sowie die Position der Kegelspitze.

Liste der Bezugs- und Formelzeichen

| | |
|---|---|
| 10 | Werkstück |
| 12, 12' | Kugelförmige Struktur |
| 20 | Messplatz |
| 22 | punktförmige Strahlungsquelle |
| 24 | Kegelförmige Strahlung |
| 26 | Kreisförmiger Bereich |
| 28 | Detektorarray |
| 29a, b,c,d | Kanten von 28 |
| 30 | Detektoren |
| 40 | Drehtisch |
| 42 | Oberfläche |
| 44 | Drehachse |
| 46 | Pfeil |
| 47 | Ursprung des x, y, z und des x**, y*, z* Koordinatensystems |
| 50, 50' | zweidimensionales Abbild von 12 |
| 52a,b,c,52a',b',c' | Punkte auf Rand von 50, 50' |
| 53 | Verbindungslinie 22-D |
| 54, 54' | Mittelpunktsstrahlen |
| 58 | Tangentialstrahl |
| 60 | Tangentialpunkt |
| 64 | Ausgleichskörper |
| D | Mittelpunkt Detektorarray 28 |
| K | Mittelpunkt Struktur 12 |
| $K_A$ | Mittelpunkt des Ausgleichskörpers |

| M | geometrischer Mittelpunkt Werkstück 10 |
|---|---|
| Mk, Mk' | Mittelpunkt von 50, 50' |
| R | Radius Struktur 12 |
| $R_A$ | Radius des Ausgleichskörpers |
| Rk, Rk' | Radius von 50, 50' |
| x, y, z | Koordinaten raumfestes Koordinatensystem |
| x*, y*, z* | Koordinaten raumfestes Koordinatensystem |
| $x_k^*, y_k^*, z_k^*$ | Koordinaten Mittelpunkt K im rotierenden Koordinatensystem |
| $x_m$ | x-Koordinate des Kreismittelpunktes MK, Mk' von 50, 50' |
| $z_m$ | z- Koordinate des Kreismittelpunktes MK, Mk' von 50, 50' |
| φ | Drehwinkel um 44 |

**Patentansprüche**

1. Verfahren zum tomographischen Vermessen von mechanischen Werkstücken (10), die eine rotationssymmetrische Struktur (12) aufweisen, wobei das Werkstück (10) und eine das Werkstück (10) durchdringende Strahlung (24) von einer Strahlungsquelle (22) schrittweise relativ zueinander bewegt werden, aus der Wechselwirkung von Werkstück (10) und Strahlung (24) in jeder Bewegungsstellung des Werkstücks (10, 10') ein zweidimensionales Abbild (50, 50') des Werkstücks (10, 10') in einer Abbildungsebene erzeugt und aus den zweidimensionalen Abbildern (50, 50') ein dreidimensionales Abbild der Struktur (12) errechnet wird, **dadurch gekennzeichnet, dass** aus mindestens zwei in zwei unterschiedlichen Bewegungsstellungen erzeugten zweidimensionalen Abbildern (50, 50') des Werkstücks (10, 10') an einem kontrastreichen Übergang in den zweidimensionalen Abbildern (50, 50') Punkte (52a, 52a', 52b, 52b', 52c, 52c') erfasst werden, die Kanten der Struktur (12) entsprechen können, aus der Lage der erfassten Punkte (52a, 52a', 52b, 52b', 52c, 52c') ein dreidimensionaler geometrischer Ausgleichskörper (64) bestimmt wird, und dieser Ausgleichskörper (64) mit einer vorgegebenen Soll-Struktur verglichen wird, wobei der geometrische Ausgleichskörper (64) nur einzelne rotationssymmetrische Elemente des Werkstücks (10) parametrisiert, die sich in den zweidimensionalen Abbildern (50, 50') mit scharfen, kontrastreichen Objektgrenzen abbilden.

2. Verfahren nach Anspruch 1, **dadurch gekennzeichnet, dass** die Objektgrenzen der Struktur (12) mit einer Kantenerkennung aus den zweidimensionalen Abbildern (50, 50') extrahiert werden.

3. Verfahren nach Anspruch 1 oder 2, **dadurch gekennzeichnet, dass** Tangentialpunkte (60) ermittelt werden, an denen ein Tangentialstrahl (58) der Strahlung (24), der von der Strahlungsquelle (22) zu einem der Punkte (52a, 52a', 52b, 52b', 52c, 52c') führt, die Struktur (12) berührt, wobei die Tangentialpunkte (60) für eine Best-Fit-Berechnung des Ausgleichskörpers (64) verwendet werden.

4. Verfahren nach einem der Ansprüche 1 bis 3, **dadurch gekennzeichnet, dass** die Sollstruktur eine rotationssymmetrische Struktur ist.

5. Verfahren nach Anspruch 4, **dadurch gekennzeichnet, dass** die Sollstruktur eine Kugel ist.

6. Verfahren nach Anspruch 4, **dadurch gekennzeichnet, dass** die Sollstruktur ein Zylinder ist.

7. Verfahren nach Anspruch 4, **dadurch gekennzeichnet, dass** die Sollstruktur ein Kegel ist.

8. Verfahren nach einem oder mehreren der Ansprüche 1 bis 7, **dadurch gekennzeichnet, dass** die Strahlungsquelle (22) und die Abbildungsebene für die relative Bewegung raumfest sind und dass das Werkstück (10) in vorgegebenen

Winkelschritten (φ) um eine Drehachse (44) gedreht wird.

9. Verfahren nach einem oder mehreren der Ansprüche 1 bis 8, **dadurch gekennzeichnet, dass** bei einer kugelförmigen Soll-Struktur die Koordinaten eines Mittelpunktes ($K_A$) und ein Radius ($R_A$) des Ausgleichskörpers (64) ermittelt werden.

10. Verfahren nach Anspruch 9, **dadurch gekennzeichnet, dass** zum Ermitteln der Lage des Mittelpunktes ($K_A$) des Ausgleichskörpers (64) in folgenden Schritten vorgegangen wird:

a) Ermitteln der Koordinaten von mindestens drei Punkten (52a, 52b, 52c) auf dem Rand des kreisförmigen, zweidimensionalen Abbildes (50) der Ist-Struktur (12) in einer ersten Bewegungsstellung des Werkstücks (10);
b) Ermitteln eines ersten Mittelpunktes ($M_k$) des kreisförmigen Abbildes (50) aus den Koordinaten der drei Punkte (52a, 52b, 52c);
c) Verdrehen des Werkstücks (10) einerseits und der Strahlungsquelle (22) sowie der Abbildungsebene andererseits relativ zueinander um einen vorgegebenen Winkelschritt (φ) von der ersten in eine zweite Bewegungsstellung des Werkstücks (10);
d) Ermitteln der Koordinaten von mindestens drei Punkten (52a', 52b', 52c') auf dem Rand des bewegten kreisförmigen, zweidimensionalen Abbildes (50') der Ist-Struktur (12) in der zweiten Bewegungsstellung des Werkstücks (10);
e) Ermitteln eines zweiten Mittelpunktes ($M_k$') des bewegten kreisförmigen Abbildes (50') aus den Koordinaten der drei Punkte (52a', 52b', 52c');
f) Ermitteln zweier Mittelpunktsstrahlen (54, 54') zwischen der Strahlungsquelle (22) und dem ersten bzw. zweiten Mittelpunkt ($M_k$, $M_k$');
g) Ermitteln des Ortes des geringsten Abstandes zwischen den Mittelpunktsstrahlen (54, 53');
h) Fällen des Verbindungslotes zwischen den Mittelpunktsstrahlen (54, 54') am Ort der geringsten Abstandes; und
i) Ermitteln der Koordinaten des Mittelpunktes des Verbindungslotes als Ort des Mittelpunktes ($K_A$) des Ausgleichskörpers (64).

11. Verfahren nach Anspruch 10, **dadurch gekennzeichnet, dass** zum Ermitteln des Radius ($R_A$) des Ausgleichskörpers (64) in folgenden Schritten vorgegangen wird:

j) Ermitteln eines ersten Abstandes zwischen der Strahlungsquelle (22) und dem Mittelpunkt ($M_k$) des kreisförmigen Abbildes (50);
k) Ermitteln eines zweiten Abstandes zwischen der Strahlungsquelle (22) und dem Mittelpunkt (K) der Ist-Struktur (12); und
l) Multiplizieren des Radius ($R_k$) des kreisförmigen Abbildes (50) mit dem Quotienten aus dem zweiten und dem ersten Abstand.

## Claims

1. A method for measuring mechanical workpieces (10) having a rotationally symmetric structure (12) by tomography, wherein a workpiece (10) and radiation (24) from a radiation source (22) penetrating the workpiece (10) are moved relative to one another step-by-step, wherein a two-dimensional image (50, 50') of the workpiece (10, 10') is generated in an imaging plane from the interaction of the workpiece (10) and the radiation (24) in each movement position of the workpiece (10, 10'), and wherein a three-dimensional image of the structure (12) is computed from the two-dimensional images (50, 50'), **characterized in that**, from at least two two-dimensional images (50, 50') of the workpiece (10, 10') generated at two different movement positions, points (52a, 52a', 52b, 52b', 52c, 52c') which may correspond to edges of the structure (12) are registered at a high-contrast transition in said two-dimensional images (50, 50'), a three-dimensional geometric equivalent body (64) is determined from the position of the registered points (52a, 52a', 52b, 52b', 52c, 52c'), and said equivalent body (64) is compared to a predefined nominal structure, wherein the geometric equivalent body (64) only parameterizes individual rotationally symmetric elements of the workpiece (10), which are imaged in the two-dimensional images (50, 50') with clear-cut, high-contrast object boundaries.

2. The method of claim 1, **characterized in that** the object boundaries of the structure (12) are extracted from the two-dimensional images (50, 50') by means of an edge recognition.

**3.** The method of claim 1 or 2, **characterized in that** tangent points (60) are determined, at which a tangent beam (58) of the radiation (24) leading from the radiation source (22) to one of the points (52a, 52a', 52b, 52b', 52c, 52c') touches the structure (12), and wherein the tangent points (60) are used for a best-fit calculation of the equivalent body (64).

**4.** The method of one of claims 1 to 3, **characterized in that** the nominal structure (12) is a rotationally symmetric structure.

**5.** The method of claim 4, **characterized in that** the nominal structure (12) is a sphere.

**6.** The method of claim 4, **characterized in that** the nominal structure is a cylinder.

**7.** The method of claim 4, **characterized in that** the nominal structure is a cone.

**8.** The method of one or more of claims 1 to 7, **characterized in that** the radiation source (22) and the imaging plane for the relative movement are fixed in space and **in that** the workpiece (10) is rotated about a rotational axis (44) in predefined angular steps ($\varphi$).

**9.** The method of one or more of claims 1 to 8, **characterized in that** coordinates of a center point ($K_A$) and a radius ($R_A$) of the equivalent body (64) are determined in the case of a spherical nominal structure.

**10.** The method of claim 9, **characterized in that** the following steps are followed to determine the position of the center point ($K_A$) of the equivalent body (64):

a) determining the coordinates of at least three points (52a, 52b, 52c) on the boundary of the circular, two-dimensional image (50) of the actual structure (12) in a first movement position of the workpiece (10);
b) determining a first center point ($M_k$) of the circular image (50) from the coordinates of the three points (52a, 52b, 52c);
c) rotating the workpiece (10) on the one hand, and the radiation source (22) and the imaging plane on the other hand relative to one another by a predefined angular step ($\varphi$) from the first into a second movement position of the workpiece (10);
d) determining the coordinates of at least three points (52a', 52b', 52c') on the boundary of the moved circular, two-dimensional image (50') of the actual structure (12) in the second movement position of the workpiece (10);
e) determining a second center point ($M_{k'}$) of the moved circular image (50') from the coordinates of the three points (52a', 52b', 52c');
f) determining two center beams (54, 54') between the radiation source (22) and the first and second center points ($M_k$, $M_k'$);
g) determining the position of the smallest distance between the center beams (54, 53');
h) dropping the connection perpendicular between the center beams (54, 54') at the position of the smallest distance; and
i) determining the coordinates of a center point of the connection perpendicular as the position of the center point ($K_A$) of the equivalent body (64).

**11.** The method of claim 10, **characterized in that** the following steps are followed to establish the radius ($R_A$) of the equivalent body (64):

j) determining a first distance between the radiation source (22) and the center point ($M_k$) of the circular image (50);
k) determining a second distance between the radiation source (22) and the center point (K) of the actual structure (12); and
l) multiplying the radius ($R_k$) of the circular image (50) by the ratio between the second and the first distance.

**Revendications**

**1.** Procédé de mesure tomographique de pièces mécaniques (10) qui présentent une structure (12) symétrique en rotation, dans lequel
la pièce (10) et un rayonnement (24) provenant d'une source de rayonnement (22) et traversant la pièce (10) sont déplacés par pas l'un par rapport à l'autre,

une image bidimensionnelle (50, 50') de la pièce (10, 10') étant formée dans un plan d'image à partir de l'interaction entre la pièce (10) et le rayonnement (24) dans chaque position de déplacement de la pièce (10, 10') et
une image tridimensionnelle de la structure (12) étant calculée à partir des images bidimensionnelles (50, 50'),
**caractérisé en ce que**
à partir d'au moins deux images bidimensionnelles (50, 50') de la pièce (10, 10'), formées en deux positions différentes de déplacement, des points (52a, 52a', 52b, 52b', 52c, 52c') qui peuvent correspondre aux arêtes de la structure (12) sont saisis dans une transition à fort contraste dans les images bidimensionnelles (50, 50'),
**en ce qu'**à partir de la position des points (52a, 52a', 52b, 52b', 52c, 52c') saisis, un corps géométrique tridimensionnel de compensation (64) est défini et
**en ce que** ce corps de compensation (64) est comparé à une structure de consigne prédéterminée,
le corps géométrique de compensation (64) ne paramétrisant que certains éléments symétriques en rotation de la pièce (10) qui forment dans les images bidimensionnelles (50, 50') des limites d'objet nettes et contrastées.

2. Procédé selon la revendication 1, **caractérisé en ce que** les limites d'objet de la structure (12) sont extraites des images bidimensionnelles (50, 50') à l'aide d'une reconnaissance d'arêtes.

3. Procédé selon les revendications 1 ou 2, **caractérisé en ce que** des points tangentiels (60) sur lesquels un faisceau tangentiel (58) du rayonnement (24) qui conduit de la source de rayonnement (22) à l'un des points (52a, 52a', 52b, 52b', 52c, 52c') touche la structure (12), les points tangentiels (60) étant utilisés pour un calcul du meilleur ajustement du corps de compensation (64).

4. Procédé selon l'une des revendications 1 à 3, **caractérisé en ce que** la structure de consigne est une structure à symétrie de rotation.

5. Procédé selon la revendication 4, **caractérisé en ce que** la structure de consigne est une sphère.

6. Procédé selon la revendication 4, **caractérisé en ce que** la structure de consigne est un cylindre.

7. Procédé selon la revendication 4, **caractérisé en ce que** la structure de consigne est un cône.

8. Procédé selon l'une ou plusieurs des revendications 1 à 7, **caractérisé en ce que** la source de rayonnement (22) et le plan de formation d'image sont fixés dans l'espace pour le déplacement relatif et **en ce que** la pièce (10) est tournée autour d'un axe de rotation (44) par pas angulaires ($\varphi$) prédéterminés.

9. Procédé selon l'une ou plusieurs des revendications 1 à 8, **caractérisé en ce que** lorsque la structure de consigne est sphérique, les coordonnées d'un point central ($K_A$) et un rayon ($K_A$) du corps de compensation (64) sont déterminés.

10. Procédé selon la revendication 9, **caractérisé en ce que** pour déterminer la position du point central ($K_A$) du corps de compensation (64), on procède par les étapes suivantes :

a) détermination des coordonnées d'au moins trois points (52a, 52b, 52c) sur la bordure de l'image bidimensionnelle circulaire (50) de la structure effective (12) dans une première position de déplacement de la pièce (10),
b) détermination d'un premier point central ($M_K$) de l'image circulaire (50) à partir des coordonnées des trois points (52a, 52b, 52c),
c) rotation entre la pièce (10), d'une part, et la source de rayonnement (22) ainsi que le plan de formation d'image, d'autre part, sur un pas angulaire prédéterminé ($\varphi$) depuis la première position de déplacement jusque dans une deuxième position de déplacement de la pièce (10),
d) détermination des coordonnées d'au moins trois points (52a', 52b', 52c') sur la bordure de l'image bidimensionnelle circulaire (50') déplacée de la structure effective (12) dans la deuxième position de déplacement de la pièce (10),
e) à partir des coordonnées des trois points (52a', 52b', 52c'), détermination d'un deuxième point central ($M_k$') de l'image circulaire déplacée (50'),
f) détermination de deux faisceaux (54, 54') de point central entre la source de rayonnement et le premier et le deuxième point central ($M_k$, $M_{k'}$)
g) détermination du lieu de la plus petite distance entre les faisceaux (54, 54') de point central,
h) projection de la verticale de liaison entre les faisceaux (54,54') de point central sur le lieu de la plus petite distance et

i) détermination des coordonnées du point central de la verticale de liaison en tant que lieu du point central ($K_A$) du corps de compensation (64).

11. Procédé selon la revendication 10, **caractérisé en ce que** pour déterminer le rayon ($R_A$) du corps de compensation (64), on procède par les étapes ci-dessous :

j) détermination d'une première distance entre la source de rayonnement (22) et le point central ($M_k$) de l'image circulaire (50),

k) détermination d'une deuxième distance entre la source de rayonnement (22) et le point central (K) de la structure effective (12) et

l) multiplication du rayon ($R_K$) de l'image circulaire (50) par le quotient entre la première et la deuxième distance.

Fig.1A

Fig.1B

Fig.1C

13

Fig.2A

Fig.2B

Fig.3A

Fig.3C

Fig.3B

EP 2 263 075 B1

Fig.4.

Fig.5

**IN DER BESCHREIBUNG AUFGEFÜHRTE DOKUMENTE**

**In der Beschreibung aufgeführte Patentdokumente**

- DE 102005039422 A1
- US 6341153 B1

- DE 102005032687 A1
- DE 3924066 A1